(19) Europäisches Patentamt

European Patent Office

Office européen des brevets

(11) Publication number: **0 403 672 B1**

(12) **EUROPEAN PATENT SPECIFICATION**

(45) Date of publication of patent specification: **29.12.93**

(51) Int. Cl.5: **C07C 15/44**, C07C 7/20

(21) Application number: **89111215.3**

(22) Date of filing: **20.06.89**

(54) **Method of inhibiting polymerization of styrenes.**

(43) Date of publication of application:
**27.12.90 Bulletin 90/52**

(45) Publication of the grant of the patent:
**29.12.93 Bulletin 93/52**

(84) Designated Contracting States:
**BE DE FR GB IT NL**

(56) References cited:
FR-A- 2 217 301
GB-A- 2 056 481
US-A- 2 181 102

(73) Proprietor: **MITSUBISHI PETROCHEMICAL CO., LTD.**
**5-2, Marunouchi 2-chome**
**Chiyoda-ku Tokyo(JP)**

Proprietor: **HÜLS AKTIENGESELLSCHAFT**

**D-45764 Marl(DE)**

(72) Inventor: **Takahashi,Hideyuki**
**c/o Mitsubishi Petrochem Co.Ltd**
**8-3-1**
**Chuo Ami-machi Inashiki-gun Ibaraki(JP)**
Inventor: **Suzuki,Shohei**
**c/o Mitsubishi Petrochem Co.Ltd**
**1,Toho-cho Yokkaichi-shi Mie(JP)**
Inventor: **Takahama,Tomohiko**
**c/o Mitsubishi Petrochem Co.Ltd**
**1,Toho-cho Yokkaichi-shi Mie(JP)**
Inventor: **Aoki,Tadamichi**
**c/o Mitsubishi Petrochem Co.Ltd**
**1,Toho-cho Yokkaichi-shi Mie(JP)**
Inventor: **Higaki,Yoshikazu**
**c/o Mitsubishi Petrochem Co.Ltd**
**1,Toho-cho Yokkaichi-shi Mie(JP)**
Inventor: **Trukenbrod,Karl**
**Leunaer Strasse 32**
**D-4370 Marl(DE)**

(74) Representative: **Hansen, Bernd, Dr.rer.nat. et al**
**Hoffmann, Eitle & Partner**
**Patentanwälte**
**Postfach 81 04 20**
**D-81904 München (DE)**

**Description**

FIELD OF THIS INVENTION

The present invention relates to a method of inhibiting polymerization of styrenes, more particularly, to a method of inhibiting polymerization applicable during the distillation process for separating and purifying styrenes.

BACKGROUND OF THIS INVENTION

High-purity styrenes usable for industries have been conventionally produced by separating and purifying a mixture generated from reaction by distillation. Styrenes, such as styrene, a substituted styrene, divinylbenzene, etc. have property of easily being polymerized, and thus, distillation is conventionally effected in the presence of a polymerization inhibiting agent.

A wide variety of compounds have been investigated for composing an agent of inhibiting the polymerization of the styrenes. However, of those conventional agents of inhibiting the polymerization of the styrenes available today, only nitrophenols and nitrosophenols have proved to be durable for industrial use in the distillation process. When sulfur is made available for example, environmental contamination arising from the burning of distilled residue is the critical problem. On the other hand, substituted alkylphenols, such as p-tertiarybutyl catechol, have not enough ability to inhibit the polymerization of the styrenes at the distillation temperature when being used alone.

In addition, nitrophenols, such as 2,4-dinitrophenol, 2-methyl-4,6-dinitrophenol, 4-methyl-2,6-dinitrophenol, etc. have also been investigated. Nevertheless, any of these has mild activity to serve as the agent of inhibiting the polymerization of the styrenes, but instead, these respectively function as the polymerization retarding agent, and thus, any of these must be used with fairly high concentration. Considering toxicity incurring to human body, the nitrophenols cannot be determined as an ideal agent suited for inhibiting polymerization of the styrenes.

On the other hand, nitrosophenols have lower toxicity than that of the nitrophenols. Furthermore, according to JP-B-55-37974 (the term "JP-B" as used herein means an "examined Japanese pagent publication"), in terms of the activity for inhibiting the polymerization of the styrenes, due to high reactivity of the nitrosophenols against an alkyl radical which causes the styrenes to generate thermal polymerization reaction and also due to high activity in inhibiting the polymerization, the nitrosophenols can effectively suppress the thermal polymerization of the styrenes.

Nevertheless, due to high reactivity of the nitrosophenols against the thermally generated alkyl radical, i.e., due to high activity for inhibiting the polymerization of the styrenes, after fully inhibiting the polymerization of the styrenes, a certain polymerization similar to the thermal polymerization in the absence of the inhibiting agent may be generated, and thus, in order to use it on the industrial basis, extreme precision is required for controlling the concentration of the polymerization inhibiting agent and the operating factors, such as temperature, pressure, etc. related to the distillation system.

SUMMARY OF THIS INVENTION

After following up concentrative study on the ideal agent of inhibiting the polymerization of the styrenes capable of generating high performance in inhibiting the polymerization and retarding the polymerization even after implementing the function to inhibit the polymerization, inventors have eventually achieved the present invention. Concretely, this invention provides the improved method of inhibiting the polymerization of the styrenes comprising the process of jointly using, 2-methyl-4-nitrosophenol and p-tertiarybutyl catechol for composing the agent of inhibiting the polymerization of the styrenes usable during the distillation process of the styrenes.

DETAILED DESCRIPTION OF THIS INVENTION

Based on 2-methyl-4-nitrosophenol having sufficient functional advantage in inhibiting the polymerization of the styrenes, inventors examined the effect of combining 2-methyl-4-nitrosophenol with other polymerization inhibiting agents. To our astonishment, a variety of compounds generated the negative effect. Sulfur, N-nitrosodiphenylamine, etc. generated the negative effect when being combined with 2-methyl-4-nitrosophenol . Phenothiazine proved to be totally ineffective.

2

On the other hand, when being combined with 2-methyl-4-nitrosophenol , p-tertiarybutyl catechol (hereinafter called p-TBC) distinctly showed the positive effect, although p-TBC proved to be insufficient in inhibiting the polymerization of the styrenes when being used alone. The improved performance in inhibiting the polymerization of the styrenes obviously exceeds the range of the additive property, and yet, it is likely that synergism is generated.

The term "styrenes" as used herein means the easily-polymerizable aromatic vinyl compounds, such as styrene, a substituted styrene, divinylbenzene.

Any optional weight ratio is available for combining p-TBC with 2-methyl-4-nitrosophenol . However, it is desired that p-TBC be used in an amount of from 0.1 to 2.0, preferably from 0.2 to 1.2, part by weight based on 1.0 part by weight of 2-methyl-4-nitrosophenol . If both of these were used less than the recommended range, then the synergism lowers. Conversely, if both of these were used more than the recommended range, then, it will result in the economic disadvantage.

Normally, the styrenes, such as styrene for example, are generated by dehydrogenation from ethylbenzene. Reaction mainly generates a mixture of styrene and ethylbenzene, which are then separated and purified by the distillation process. Conventionally, the styrenes are distilled under reduced condition, at the temperature from 70 to 130°C with about from 8,0 to 26,7 kPa (60 to 200 mmHg) of the distillation pressure. Unless the occurrence of thermal polymerization is prevented in the above temperature range, large volume of polymer is generated.

2-methyl-4-nitrosophenol is generally used in an amount of from 50 to 300, preferably from 50 to 150, ppm based on the amount of the styrenes.

If 100 ppm of the polymerization inhibiting agent embodied by this invention on the basis of 2-methyl-4-nitrosophenol fed to the distillation process were present against such polymerizable monomer, such as styrene, then, the polymerization inhibiting agent can fully generate the own effect for inhibiting the polymerization of the styrenes. Furthermore, the polymerization inhibiting agent embodied by this invention can be fed to the distillation system through the following processes: First, the powdered 2-methyl-4-nitrosophenol and p-TBC are fed in the blended or independent condition to the dissolving pool provided for the preparatory process. Then both are dissolved using crude styrene, containing ethylbenzene. Finally, the dissolved compound is fed to the distillation tower. Instead of crude styrene, etc., inert solvent capable of dissolving the polymerization inhibiting agent may also be used.

This invention has materialized an effective combination of 2-methyl-4-nitrosophenol having prominent effect and high activity for inhibiting the polymerization of the styrenes with p-TBC which does not provide sufficient effect for inhibiting the polymerization of the styrenes at the distilling temperature when being used alone. As a result, by effectively utilizing the prominent function of 2-methyl-4-nitrosophenol for inhibiting the polymerization of the styrenes, this invention provides an ideal polymerisation inhibiting agent which securely prevents occurrence of the polymerization even after inhibiting the polymerization, and thus, the agent of inhibiting the polymerization of the styrenes embodied by this invention deserves attention of all the concerned. In addition, it renders effective service for stably and continuously operating those plants producing easily-polymerizable styrenes for a long while to come.

By presenting some example and comparative examples, the method of inhibiting the polymerization of the styrenes embodied by this invention is more concretely described below.

At the following example and comparative examples, the commercially procured styrene was washed with the alkaline aqueous solution to remove the polymerization inhibiting agent used for storage, and after washing it with pure water, the prepared styrene was distilled and purified immediately before starting the experiments.

EXAMPLE

First, each 100 ppm of 2-methyl-4-nitrosophenol and p-tertiarybutyl catechol were added to the purified styrene, and then, considering the styrene distillation condition, it was determined that the tests be conducted at 110°C.

Under argon atmosphere, the blending bath temperature was raised to 110 ± 0.5°C and the blended material was continuously stirred. Then, the stirred material was sampled from time to time, and then the sampled material was analyzed by means of gel permeation chromatography (GPC) to eventually obtain the proportion of polymer in the styrene monomer (% by weight). The result is shown in Table 1.

3

COMPARATIVE EXAMPLE 1

The same procedure as for the above example was repeated except for the addition of 100 ppm of p-TBC to the purified styrene without adding 2-methyl-4-nitrosophenol. The test resulted in the generation of excessive volume of polymer, and as a result, after two hours are past, the proportion of polymer could not be eventually obtained. Test result is also shown in Table 1.

COMPARATIVE EXAMPLE 2

The same procedure as for the above example was repeated except for the addition of 100 ppm of 2-methyl-4-nitrosophenol to the purified styrene without adding p-TBC. The result is also shown in Table 1.

COMPARATIVE EXAMPLE 3

The same procedure as for the above example was repeated except for adding 600 ppm of sulfur to the purified styrene instead of 100 ppm of p-TBC. The result is also shown in Table 1.

COMPARATIVE EXAMPLE 4

The same procedure as for the above example was repeated except for adding 200 ppm of phenothiazine to the purified styrene instead of 100 ppm of p-TBC. The result is also shown in Table 1.

COMPARATIVE EXAMPLE 5

The same procedure as for the above example was repeated except for adding 300 ppm of N-nitrosodiphenylamine (NDPA) to the purified styrene instead of 100 ppm of p-TBC. The result is also shown in Table 1.

## Table 1

| Combination of polymerization inhibiting agent | Proportion of generated polymer (% by weight) | | | |
| --- | --- | --- | --- | --- |
| | 1 hour | 2 hours | 3 hours | 4 hours |
| Example — Each 100 ppm of 2-methyl-4-nitrosophenol and p-TBC | 0.0 | 0.13 | 0.25 | 0.69 |
| Comparative Example 1 — 100 ppm of p-TBC | 2.0 | (Measurement impossible) | (Measurement impossible) | (Measurement impossible) |
| Comparative Example 2 — 100 ppm of 2-methyl-4-nitrosophenol | 0.0 | 0.19 | 0.42 | 1.29 |
| Comparative Example 3 — 100 ppm of 2-methyl-4-nitrosophenol and 600 ppm of sulfur | 0.1 | 1.30 | 2.69 | 4.09 |
| Comparative Example 4 — 100 ppm of 2-methyl-4-nitrosophenol and 200 ppm of phenothiazine | 0.0 | 0.20 | 0.41 | 1.22 |
| Comparative Example 5 — 100 ppm of 2-methyl-4-nitrosophenol and 300 ppm of NDPA | 0.1 | 1.27 | 2.51 | 3.81 |

## Claims

1. A method of inhibiting polymerization of styrenes comprising the process of jointly using 2-methyl-4-nitrosophenol and p-tertiarybutyl catechol for composing the polymerization inhibiting agent usable during the distillation process of styrenes.

2. The method as claimed in claim 1, wherein said styrenes are selected from the group consisting of styrene, a substituted styrene and divinylbenzene.

3. The method as claimed in claim 1, wherein p-tertiarybutyl catechol is used in an amount of from 0.1 to 2.0 parts by weight based on 1.0 part by weight of the 2-methyl-4-nitrosophenol.

4. The method as claimed in claim 1, wherein 50 to 300 ppm of the 2-methyl-4-nitrosophenol are present against the styrenes.

5. The method as claimed in claim 1, wherein the styrenes are distilled at the temperature from 70 to 130°C.

**Patentansprüche**

1. Verfahren zur Inhibierung der Polymerisation von Styrolen, das die gemeinsame Verwendung von 2-Methyl-4-nitrosophenol und p-Tertiärbutylcatechol zur Bildung des polymerisationsinhibierenden Mittels, das während des Destillationsverfahrens der Styrole einsetzbar ist, umfaßt.

2. Verfahren nach Anspruch 1, wobei die Styrole aus der Reihe Styrol, substituiertes Styrol und Divinylbenzol ausgewählt werden.

3. Verfahren nach Anspruch 1, wobei p-Tertiärbutylcatechol in einer Menge von 0,1 bis 2,0 Gewichtsteilen pro 1,0 Gewichtteil 2-Methyl-2-nitrosophenol verwendet wird.

4. Verfahren nach Anspruch 1, wobei 50 bis 300 ppm 2-Methyl-4-nitrosophenol gegenüber den Styrolen vorliegen.

5. Verfahren nach Anspruch 1, wobei die Styrole bei einer Temperatur von 70 bis 130°C destilliert werden.

**Revendications**

1. Un procédé d'inhibition de la polymérisation des styrènes comprenant le procédé d'utilisation simultanée de 2-méthyl-4-nitrosophénol et de p-tertibutylcathéchol pour composer l'agent inhibiteur de polymérisation utilisable pendant le procédé de distillation des styrènes.

2. Le procédé selon la revendication 1, dans lequel lesdits styrènes sont choisis parmi le styrène, les styrènes substitués et le divinylbenzène.

3. Le procédé selon la revendication 1, dans lequel on utilise le p-tertiobutylcatéchol en quantité de 0,1 à 2,0 parties en poids pour 1,0 partie en poids du 2-méthyl-4-nitrosophénol.

4. Le procédé selon la revendication 1, dans lequel le 2-méthyl-4-nitrosophénol est présent en quantité de 50 à 300 ppm par rapport aux styrènes.

5. Le procédé selon la revendication 1, dans lequel les styrènes sont distillés à une température de 70 à 130°C.